# DEMANDE DE BREVET EUROPEEN

(11) **EP 2 792 366 A1**
(43) Date de publication de la demande: **22.10.2014**
(21) Numéro de dépôt: 14170934.5
(22) Date de dépôt: 26.12.2006
(51) Int. Cl.: A61K 39/395, A61P 43/00, C07K 16/00, C07K 1/36

(54) **Concentre d'immunoglobulines G (IgG) appauvri en anticorps anti-A et anti-B, et en IgG polyreactives**

(30) Priorité: 26.12.2005 FR 0513311
(62) Demande divisionnaire de: 06847150.7
(71) Demandeur: Laboratoire Français du Fractionnement et des Biotechnologies, 91940 Les Ulis (FR)
(72) Inventeur: Chtourou, Abdessatar, 78990 Elancourt (FR); Dhainaut, Frédéric, 91870 Boissy-le-Sec (FR); Paolantonacci, Philippe, 91190 Gif sur Yvette (FR)
(74) Mandataire: Hirsch & Associés

(57) **Abrégé**

La présente invention concerne un concentré d'immunoglobulines G à usage thérapeutique dans lequel les teneurs respectives en anticorps anti-A et anti-B sont conformes à un résultat négatif au test de Coombs indirect in vitro. Ce concentré d'IgG présente en outre une teneur d'IgG polyréactives comprise entre 0,01% et 0,1%, en particulier entre 0,07% et 0,1%, par rapport à la teneur totale en IgG.

## Description

La présente invention se rapporte à un concentré d'immunoglobulines G (IgG) appauvri en anticorps anti-A (AcaA) et anti-B (AcaB) et de polyréactivité fortement réduite, ainsi qu'à un procédé d'obtention de tels concentrés.

L'utilisation de fractions de plasma humain enrichies en immunoglobulines (Ig) pour le traitement de diverses infections ou déficits congénitaux est connue depuis la mise au point du procédé de précipitation à l'éthanol par Cohn (Cohn et al. 1946, J. Am. Chem. Soc. 68, 459 ; Oncley et al. 1949, J. Am. Chem. Soc. 71, 541).

Il y a, à cet effet, un besoin grandissant de produire des concentrés d'Ig hautement purifiés, injectables par voie intraveineuse (IgIV), à partir par exemple de plasmas humains. La complexité de la structure des immunoglobulines (quatre chaînes polypeptidiques réunies par des ponts disulfure), la variété des anticorps présents dans le mélange du plasma de plusieurs milliers de donneurs ne sont pas actuellement des facteurs favorisant le développement biotechnologique des immunoglobulines. Bien que des anticorps monoclonaux soient produits par ingénierie génétique, leur extrême spécificité constitue un inconvénient pour les applications thérapeutiques où une polyspécificité semble nécessaire.

En outre, de nombreuses pathologies par exemple d'origine autoimmune sont actuellement traitées par des concentrés d'IgG ce qui a engendré une situation de pénurie en Europe et aux Etats Unis d'Amérique au cours de ces dernières années.

Des procédés d'obtention d'immunoglobulines et notamment de concentrés d'IgG peuvent comprendre, outre la précipitation sélective des protéines par l'éthanol, divers traitements additionnels comme la précipitation par le polyéthylène glycol, le traitement ménagé par des enzymes protéolytiques..., destinés à éliminer les agrégats de polymères d'immunoglobulines susceptibles d'activer le système du complément avec des risques associés de réactions anaphylactiques. Par ailleurs, la présence de dimères dans les IgGIV a été corrélée à des chutes de tension artérielle *in vivo* (Bleeker W.K. et al, Blood, 95, 2000, p. 1856-1861).

Une voie alternative à la précipitation par l'éthanol a été décrite par Steinbuch et al. (Rev. Franç. Et. Clin. et Biol. 1969, XIV, 1054) qui fait appel à une précipitation par l'acide octanoique. Celui-ci précipite la plupart des protéines du plasma et laisse dans le surnageant les immunoglobulines. La purification de ces immunoglobulines est réalisée par passage sur un échangeur d'anions, la DEAE-cellulose, dans des conditions qui ne retiennent pas les IgG. La fraction d'IgG non retenue est ensuite concentrée.

Divers procédés ont également été mis au point pour augmenter la pureté des produits par la mise en oeuvre de techniques chromatographiques. On peut notamment citer les demandes de brevets EP 0 703 922 et WO 99/64462, qui décrivent l'association d'au moins deux étapes successives de chromatographie, l'une par échange d'anions, l'autre par échange de cations. La spécificité de ces procédés est apportée par la propriété des échangeurs d'anions de ne pas retenir les immunoglobulines G, dans les conditions classiques de mise en oeuvre, mais de fixer la plupart des autres protéines co-purifiées au cours des étapes de prépurification. De même, on peut citer la demande de brevet WO 02/092632 déposée par la Demanderesse qui divulgue la préparation de concentrés d'Ig mettant en oeuvre une unique étape de chromatographie sur échangeur d'anions, effectuée à un pH alcalin pour leur rétention sur le support chromatographique.

Cependant, de nombreuses publications scientifiques indiquent que l'injection d'IgG obtenues par les techniques de fractionnement ci-dessus peuvent provoquer des hémolyses accidentelles, dont certaines sévères, chez des patients en traitement. On peut à titre d'exemple citer les publications de Buchta C. et al, Biologicals, 33, 2005, 41-48, Wilson J.R. et al, Muscle & Nerve, 29(9), 1997, 1142-1145, Copelan E.A et al, Transfusion, 26, 1986, 410-412 et Misbah S.A et al, Drug Safety, 9, 1993, 254-262. L'étude des effets sur le sang des patients présentant une hémolyse, mise en oeuvre notamment par le test de Coombs direct (DCT), a montré que les hématies sont couvertes d'immunoglobulines dirigés contre les antigènes A, B ou D présents à leur surface provoquant ainsi leur hémolyse. C'est ainsi que les IgG disponibles dans le commerce sont obtenues à partir de plasmas sélectionnés pour éviter la présence d'immunoglobulines anti-D ou d'autres anticorps à forts titres en anti-A ou anti-B.

Buchta C. *et al*, cité plus haut, a envisagé différentes approches pour réduire de façon significative les anticorps anti-A, provenant de donneurs de groupes sanguins B et O, et anti-B, provenant de donneurs de groupes sanguins A et O, dans les dérivés de plasma, tels que les IgG, pour ainsi minimiser les risques d'hémolyse, directement corrélés aux taux de ces anticorps, lors du traitement des patients par ces dérivés du plasma. Il est notamment envisagé de choisir les donneurs, d'éliminer les anticorps anti-A et anti-B, de fabriquer des dérivés du plasma sanguin issu de groupe spécifique, de groupes A et/ou B, ou de ne pas inclure dans les lots de plasmas de titre élevé en anticorps anti-A ou anti-B. Certaines approches sont considérées non réalistes en raisons des coûts ou de la complexité des mesures à prendre. On note que les anticorps anti-A et anti-B sont partiellement éliminés au cours du fractionnement éthanolique mentionné ci-dessus.

Etant donné que les besoins en IgG sont en constante augmentation, il est nécessaire de disposer de pools de plus en plus importants de donneurs qui seront statistiquement d'autant plus riches en groupe sanguin O. En conséquence, les dérivés sanguins, tels que les IgG, contiennent des quantités d'anticorps anti-A et anti-B trop élevées pour être éliminées par le fractionnement classique.

Ces besoins croissants ne rendent pas non plus envisageable de sélectionner par exemple uniquement les donneurs de groupes AB pour s'assurer d'une faible teneur en anticorps anti-A et anti-B.

Chaque lot de concentrés ou de préparations d'IgG purifiées est contrôlé vis-à-vis des anticorps anti-A et anti-B selon le test de la Pharmacopée Européenne 2.6.20 (1997), lequel est une application du test de Coombs indirect (ICT) in vitro. L'essai ICT consiste à ajouter à une suspension d'hématies, revêtues des anticorps anti-A ou anti-B de type IgG contenus dans les concentrés d'IgG, une solution d'anticorps (antiglobulines) dirigés, contre des motifs de l'IgG humaine. Ces anticorps se fixent sur des anticorps anti-A ou anti-B attachés aux hématies et permettent ainsi leur agglutination par formation de ponts entre les IgG. L'essai de recherche d'anticorps anti-A ou anti-B s'inspire directement de ce test classique en sérologie hématologique (test de Coombs).

Selon la Pharmacopée Européenne, les IgIV ne doivent pas présenter d'agglutination des globules rouges A ou B à l'essai ICT à la dilution 1:64 mis en oeuvre avec une solution d'IgG de concentration initiale ramenée à 30 g/l.

C'est la raison pour laquelle il convient de diluer les échantillons d'IgG à tester pour obtenir un titre, c'est-à-dire la valeur de la dernière dilution ne provoquant plus d'agglutination. Des résultats négatifs au test ICT sur les solutions d'IgIV dont les dilutions sont inférieures à la dilution 1:64, selon la Pharmacopée Européenne démontrent un faible taux en anticorps anti-A et anti-B acceptés par celle-ci. Toutefois, même avec des concentrés d'IgG donnant un résultat négatif au test prescrit par la Pharmacopée Européenne, c'est-à-dire ceux dont le taux de dilution est inférieur à 1:64, les risques de réactions hémolytiques ne peuvent être exclus (Buchta *et al* déjà cité).

Il convient de noter que les Pharmacopées américaine et japonaise ne contiennent aucune disposition sur la nécessité de contrôler les teneurs résiduelles en anticorps anti-A et anti-B.

Comme mentionné plus haut, les anticorps anti-A et anti-B sont partiellement éliminés au cours de la préparation de concentrés d'IgG par fractionnement éthanolique mais on en observe une teneur résiduelle qui peut être supérieure à la limite des normes hautes de la Pharmacopée Européenne. De plus, les concentrés préparés selon le procédé mis au point par la Demanderesse et décrit dans sa demande de brevet WO 02/092632 en contiennent davantage que ceux obtenus par le fractionnement à l'éthanol. Une purification supplémentaire des concentrés d'IgG ainsi obtenus vis-à-vis des AcaA et AcaB est par conséquent nécessaire, étant donné que certains lots de concentrés d'IgG peuvent présenter des teneurs supérieures au seuil admis par la Pharmacopée Européenne pour chacun d'entre eux.

Une des techniques pour les éliminer des concentrés d'IgG consiste à mettre en oeuvre une purification par chromatographie d'affinité utilisant comme support des immunoadsorbants de type oligosaccharides similaires aux antigènes A et B des groupes sanguins, de tels oligosaccharides représentant en particulier des trisaccharides, greffés sur une matrice chromatographique.

A titre d'exemple, on peut citer la publication de Mazid M.A et al, J.Appl.Biomater., 3(1), 1992, 9-15, qui utilise un support chromatographique à base de particules de silice sur lesquelles sont greffées des haptènes d'oligosaccharides de synthèse caractéristiques des groupes sanguins A et B, en particulier les A-trisaccharides. Egalement, Hout M.S et al (ASAIO J, 46(6), 2000, 702-706) décrit la mise en oeuvre de membranes fibreuses tubulaires greffées d'antigènes spécifiques anti-A et anti-B pour l'élimination des anticorps anti-A et anti-B du sang total. Il est également rapporté que de tels supports chromatographiques sont très stables, ce qui limite le relargage de ces haptènes résiduels dans les concentrés d'intérêt.

La demande de brevet WO 01/27623 décrit un procédé d'obtention d'un plasma déspécifié en anticorps de groupes sanguins A et B, c'est-à-dire un plasma convenant à tout receveur. Ces spécificités sont essentiellement portées par des immunoglobulines M (IgM). La déspécification est obtenue par passage sur un support d'affinité expérimental pour le groupe A puis sur un autre support d'affinité, également expérimental, pour le groupe B. En cas de présence simultanée d'anti-A et d'anti-B (groupe O), le passage successif sur les deux supports est nécessaire.

Une des caractéristiques supplémentaires des concentrés d'IgG disponibles dans le commerce est leur polyréactivité. Il convient de rappeler que les anticorps polyclonaux tels que les IgG sont normalement combinés à un seul épitope (motif antigénique) de façon unique. Cependant, cette spécificité stricte des anticorps peut quelques fois s'élargir à d'autres motifs antigéniques et présenter une affinité pour des épitopes secondaires avec une liaison toutefois plus faible que pour le motif nominal. Les IgG polyclonales peuvent réagir plus ou moins fort avec des structures telles que l'actine, la myosine, l'albumine modifiée par le trinitrophényle.

A ce titre, les immunoglobulines intraveineuses (IgIV) sont des préparations d'IgG humaines polyclonales contenant :
- des anticorps immuns dirigés contre des antigènes externes et résultant d'un processus d'immunisation,
- des anticorps naturels reconnaissant des protéines intracellulaires, des antigènes membranaires de surface, des protéines du soi circulantes et la région variable d'autres anticorps. Ces derniers sont appelés anticorps anti-idiotypiques (Kazatchkine M.D. et al, Immunol. Rev., 139, 1994, 79-107.

Les anticorps naturels ne résultent pas d'une immunisation délibérée (Coutinho A. et al, Curr. Opin. Immunol., 7, 1995, 812-818. Ils sont polyréactifs dans le sens où ils expriment des affinités variables pour les antigènes du soi (Berneman A. et al, Eur. J. Immunol., 22, 1992, 625-631 et Lacroix-Desmazes et al, J. Immunol. Methods, 216, 1988, 117-137.

Des traitements chimiques (urée 6M, thiocyanate de sodium 1,3 M et traitement acide pH = 2,0) des IgIV peuvent augmenter l'activité polyréactive de ces immunoglobulines polyclonales (Bouvet J.P. et al, J. Autoimmun., 16(2), 2001, 163-172. Cependant, il n'a jamais été démontré d'effet bénéfique sur des patients traités au moyen de telles immunoglobulines.

En résumé, l'activité polyréactive des anticorps présents dans une préparation d'IgIV peut être due à :
- la présence d'anticorps naturels contenus dans chaque plasma individuel,
- la présence d'anticorps anti-idiotypiques contenus dans chaque plasma individuel,
- la polyréactivité des anticorps générée par le procédé de fabrication.

Ainsi, certains auteurs considèrent que la polyréactivité est une propriété intrinsèque des IgG qui sont donc naturellement présentes dans l'organisme humain.

D'autres démontrent qu'un procédé de purification des IgG monoclonales ou polyclonales peut faire apparaître une polyréactivité indécelable avant purification. En effet, les procédés de fabrication des IgG à partir du plasma engendrent également une polyréactivité se traduisant par un « stress » oxydatif et la carbonylation partielle des IgG au cours de leur mise en oeuvre.

Ceci est confirmé par Bouvet J.-P. et al, Journal of Autoimmunity, 16, 2001, pp.163-172, pour qui les IgG polyclonales deviennent fortement polyréactives après traitement à l'urée notamment. Il est également indiqué dans ce document qu'une partie de l'efficacité des IgG en utilisation clinique est attribuée à leur polyréactivité.

Il ressort que la polyréactivité de ces concentrés d'IgG peut donc s'expliquer par la présence conjointes d'IgG polyréactives naturelles et de celles polyréactives obtenues par les procédés de purification habituels, et représentent, selon les cas, 0,5 à 1% de l'ensemble des IgG polyvalentes. Le traitement de patients par les préparations d'IgG peut nécessiter d'en administrer de fortes doses, jusqu'à 1 à 2 g/kg. Ces posologies conduisent à traiter en un temps court, par exemple en une journée, par des quantités de 7 à 10 fois supérieures à celles des IgG physiologiques du receveur. Par conséquent, ce taux d'IgG polyréactives générées par le procédé de fabrication dans les concentrés d'IgG est susceptible d'engendrer des effets secondaires indésirables, telles que des fièvres, nausées ou céphalées.

Ainsi, il convient de faire une distinction entre la polyréactivité des anticorps due aux anticorps naturels et anti-idiotypiques, qui, comme l'a montré la Demanderesse dans sa demande de brevet EP 1 059 088, présente des avantages, de la polyréactivité des anticorps générée par le procédé de fabrication. En effet, la Demanderesse a montré dans sa demande de brevet EP 1 059 088 que des fractions d'IgG polyréactives contenues naturellement dans le plasma, isolées à partir d'IgGIV polyvalentes humaines, peuvent être avantageusement utilisables pour le traitement de certaines maladies inflammatoires, telle que la polyarthrite rhumatoïde, en raison d'une moindre posologie requise pour cette fraction.

Par conséquent, pour éviter des réactions indésirables tant sur le plan de l'hémolyse des hématies que sur le plan des réactions secondaires susceptibles d'être observées par l'administration massive des IgG au cours d'un traitement, il apparaît nécessaire de disposer de concentrés d'IgG à usage thérapeutique, en particulier pour injection intraveineuse, appauvris de manière significative en anticorps anti-A et anti-B et qui soient, de préférence, de polyréactivité générée par le procédé de fabrication très réduite par rapport aux concentrés d'IgG actuellement disponibles dans le commerce, tout en ayant une efficacité au moins identique sur le plan de l'immunothérapie.

Par conséquent, l'invention concerne un concentré d'immunoglobulines G à usage thérapeutique, caractérisé en ce qu'il présente des teneurs respectives en anticorps anti-A et anti-B conformes à un résultat négatif au test de Coombs indirect in vitro.

L'invention se rapporte également à un procédé de fabrication d'Immunoglobulines G permettant de ne pas générer ces anticorps polyréactifs qui peuvent être moins bien tolérés que les anticorps naturels et anti-idiotypiques. De ce fait les IgIV obtenues par le procédé ont une polyréactivité plus faible que les autres IgIV étudiées.

Dans le cadre de l'invention, les IgG de ces concentrés représentent avantageusement les IgG polyclonales obtenues à partir du plasma sanguin ou d'une fraction de plasma sanguin déjà enrichie en IgG. Les concentrés d'IgG à usage thérapeutique sont à des concentrations en IgG communément mises en oeuvre actuellement, comprises de préférence entre 50 et 100 g/l. Ces concentrés sont destinés à un usage clinique et peuvent en particulier être injectés par voie intraveineuse. A cet effet, ils doivent être viralement sécurisés et, le cas échéant, contenir des excipients, tels que des stabilisants, compatibles avec cet usage clinique.

La Demanderesse a trouvé qu'il était possible de mettre à disposition de tels concentrés d'IgG présentant des teneurs en anticorps anti-A et anti-B nettement inférieures à celles observées dans les concentrés d'IgG standards, c'est-à-dire à ceux obtenus par fractionnement éthanolique et/ou par la mise en oeuvre des techniques de purification associant des chromatographies, comme mentionné plus haut, et qui n'ont pas fait l'objet d'un traitement complémentaire d'élimination des anticorps considérés. Egalement, leurs teneurs sont nettement inférieures aux seuils acceptés par la Pharmacopée Européenne, ce qui limite très significativement les risques d'hémolyse chez certains receveurs en traitement. Lorsque l'on soumet les concentrés d'IgG de l'invention à des essais destinés à évaluer les quantités des AcaA et AcaB, on observe que les résultats des essais d'agglutination in vitro des globules rouges A, B et/ou AB en présence d'anticorps anti-IgG humaines sont systématiquement négatifs, notamment à la concentration initiale de 30 g/l imposée par la méthode de la Pharmacopée Européenne. La mise en oeuvre du test de Coombs indirect, défini précédemment, avec des concentrés d'IgG de l'invention conduit donc à des résultats systématiquement négatifs et ce même avec des échantillons d'IgG en tant que tels, non dilués. Il apparaît donc que la teneur en ces anticorps anti-A et anti-B dans ces concentrés n'est déjà pas détectable par le test ICT.

Lorsque le taux d'anticorps anti-A et anti-B est très faible dans les concentrés d'IgG, le test ICT ne peut plus s'appliquer, a fortiori dans les conditions de la Pharmacopée Européenne, étant donné que les réactions d'agglutination des hématies n'ont plus lieu, même avec l'ajout d'anticorps anti-IgG humaines, car la densité d'anticorps anti-A et anti-B est trop faible pour l'établissement de pontages entre les hématies par des liaisons anticorps anti-A et anti-B fixés sur les hématies et les anticorps anti-IgG humaines.

Il est cependant possible de vérifier la présence des ces anticorps anti-A et/ou anti-B à très faible concentration en provoquant l'immunohémolyse des globules rouges ayant fixé ces anticorps et de mesurer un appauvrissement par rapport à un concentré classique n'ayant pas subi un traitement d'élimination des AcaA et/ou AcaB. L'immunohémolyse est une réaction immunologique spécifique qui se produit lorsque l'anticorps est attaché à sa cible en présence de facteurs du complément. L'activation du système du complément aboutit à la libération de perforines qui transpercent la membrane du globule rouge, laissant sortir l'hémoglobine. Il suffit alors de mesurer par une méthode sensible, par exemple au moyen de traceurs radioactifs (voir plus loin), la quantité d'hémoglobine libérée, proportionnelle à la quantité d'anticorps anti-A et/ou anti-B présente.

La Demanderesse a montré de manière particulièrement avantageuse que le concentré d'IgG de l'invention comprend une teneur en anticorps anti-A non supérieure à 23 ng/mg d'IgG, en particulier comprise entre 19 et 23 ng/mg d'IgG, et une teneur en anticorps anti-B non supérieure à 20 ng/mg d'IgG, en particulier comprise entre 12 et 20 ng/mg d'IgG.

Avantageusement, la Demanderesse a trouvé qu'il était également possible de mettre à disposition de tels concentrés d'IgG à très faible teneur en IgG polyréactives notamment celles générées par le procédé de fabrication leur conférant ainsi un caractère quasiment non polyréactif, tout en étant aussi efficace dans le traitement des immunothérapies que les concentrés d'IgG de l'art antérieur. Cette absence notable du caractère de polyréactivité des IgG dû au procédé de fabrication réduit sensiblement les risques d'effets secondaires susceptibles d'être engendrés à la suite de traitements nécessitant de fortes posologies.

Il est également avantageusement possible de corriger les effets indésirables liés à la présence d'IgG polyréactives dues au procédé de fabrication dans le concentré d'IgG engendrée notamment par le « stress » oxydatif et la carbonylation durant les procédés de purification.

De préférence, la teneur d'IgG polyréactives résiduelle est comprise entre 0,01% et 0,1%, en particulier entre 0,07 et 0,1%. Dans le cadre de l'invention, la teneur d'IgG polyréactives signifie un pourcentage molaire ou massique. Cette teneur est déterminée par les méthodes décrites par la Demanderesse dans la demande de brevet 1 059 088.

Les concentrés d'IgG selon l'invention sont donc définis par une absence notable des principes actifs anticorps anti-A et anti-B qui sont dirigés contre les épitopes présents sur les globules rouges.

Les concentrés d'IgG peuvent se présenter sous forme liquide ou lyophilisée en présence de stabilisants appropriés, et être stockés dans l'attente d'une utilisation ultérieure. Ces stabilisants représentent avantageusement ceux mis au point par la Demanderesse dans sa demande de brevet WO 2004/091656 A2, à savoir un mélange d'un sucre alcoolique, de préférence le mannitol, le sorbitol ou leurs isomères, de glycine et d'un détergent non ionique, tel que le Tween^{®}80, le Tween^{®}20, le Triton^{®} X 100 ou le Pluronic^{®}F68, tous trois composés acceptables sur le plan pharmaceutique.

Les concentrations de la formulation ont été déterminées par la Demanderesse pour stabiliser les formes liquides et/ou lyophilisées.

De préférence, les concentrations finales dans les concentrés en mannitol sont comprises entre 30 g/l et 50 g/l, celles du détergent, entre 20 et 50 ppm, et celles de la glycine, entre 7 g/l et 10 g/l. Les concentrations de ces composés représentent les concentrations finales dans les concentrés d'IgG.

De tels concentrés d'IgG, à usage thérapeutique, peuvent en particulier être injectés par voie intraveineuse, comme indiqué précédemment. A cet effet, les concentrés d'IgG selon l'invention doivent être viralement sécurisés par exemple par un traitement classique au solvant-détergent connu dans l'art antérieur, utilisant par exemple un mélange Tween^{®}80/TnBP ou Triton^{®} X 100 /TnBP, et/ou subir des étapes de filtration pour éventuellement éliminer les virus et/ou autres macromolécules qui n'auraient pas été éliminés par le traitement virucide de solvant-détergent, tels que le prion, agent responsable des encéphalopathies spongiformes transmissibles.

L'invention concerne également un procédé d'obtention d'un concentré d'IgG tel que mentionné ci-dessus, comprenant les étapes suivantes de :
a) préparation d'un concentré d'IgG par fractionnement éthanolique et/ou par séparation chromatographique, associant une étape d'inactivation virale,
b) chromatographie d'immunoaffinité par percolation dudit concentré d'IgG sur un mélange de supports dont les matrices sont greffées de groupes oligosaccharides antigéniquement similaires aux groupes sanguins A et B, et
c) filtration d'élimination de virus et/ou de particules de taille supérieure à 20 nm.

La Demanderesse a trouvé de manière surprenante que non seulement un tel procédé pouvait très avantageusement être mis en oeuvre à l'échelle industrielle, mais que la combinaison d'étapes aboutissant à la préparation de concentrés d'IgG et d'une étape spécifique d'élimination d'anticorps anti-A et anti-B permettait d'obtenir un concentré d'IgG selon l'invention, à usage thérapeutique, comprenant en outre, de préférence, un taux d'IgG polyréactives inférieur à 0,1% par rapport à la teneur totale en IgG. De surcroît, un tel concentré comprend un taux en AcaA et AcaB indésirables bien inférieur à la valeur limite basse de l'essai décrit dans la Pharmacopée Européenne et même donnant un résultat négatif par la mise en oeuvre du test ICT avec de tels échantillons non dilués.

De préférence, l'étape a) du procédé peut être en elle-même un procédé d'obtention de concentrés d'IgG, tels que ceux mentionnés précédemment. Il s'agit d'un fractionnement éthanolique développé par Cohn *et al* ou bien d'une séparation chromatographique, telle que décrite par exemple dans EP 0 703 922 et WO 99/64462. On préfère tout particulièrement les procédés mis au point par la Demanderesse dans les demandes de brevet WO 94/29334 et WO 02/092632 A1, et tout particulièrement celui décrit dans WO 02/092632 A1. Dans ce cas, l'étape a) du procédé de l'invention comprend une prépurification par précipitation de contaminants lipidiques à partir d'un plasma sanguin ou d'une fraction de plasma sanguin enrichie en IgG, une chromatographie unique sur un support de résine échangeuse d'anions effectuée à pH alcalin, une élution sélective des IgG en une étape par un tampon approprié à pH compris entre 4 et 7.

L'étape a) du procédé comprend un traitement d'inactivation de virale, de préférence effectué par solvant-détergent, comme décrit par Horowitz dans le brevet US 4 764 369. Il sera en particulier judicieusement mis en oeuvre, le cas échéant, avant une ou l'étape chromatographique subséquente permettant d'éliminer notamment les résidus chimiques de ce traitement.

La fraction d'IgG ainsi récoltée est déjà suffisamment concentrée, et peut ensuite subir des étapes de concentration supplémentaire par ultrafiltration et de filtration stérilisante.

Ce concentré est ensuite soumis à une étape chromatographique d'immunoaffinité sur un mélange de deux supports greffés de groupes antigéniquement similaires aux groupes sanguins A et B, de préférence sur une colonne remplie d'un tel mélange de supports.

De préférence, le support chromatographique est constitué d'une matrice en polymère naturel réticulé, de type agarose, sur laquelle sont greffés des espaceurs ou des bras de couplage, étant à leur tour greffés, avec des oligosaccharides représentant avantageusement des trisaccharides correspondants aux épitopes des groupes sanguins A et B. En particulier, de très bons résultats sont obtenus en mettant en oeuvre un tel support dont les trisaccharides, correspondant à l'épitope du groupe sanguin A, présentent la structure N-acétylgalactosamine (GalNAc)-Galactose (Gal) - Fucose (Fuc), et ceux correspondant à l'épitope du groupe sanguin B, présentent la structure Galactose-Galactose-Fucose Un tel support représente très avantageusement un gel ou résine disponible dans le commerce sous la dénomination GLYCOSORB ABO^{®}, provenant de chez Glycorex Transplantation AS (Suède).

A titre d'exemple, si ce support est utilisé, le trisaccharide correspondant à l'épitope du groupe sanguin A présente la structure ci-dessous :

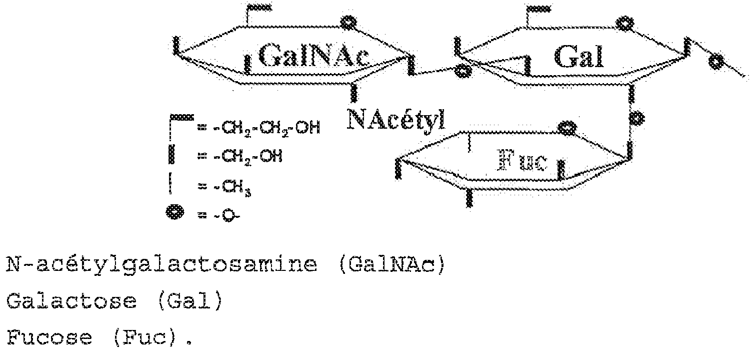

A titre d'exemple, si ce support est utilisé, le trisaccharide correspondant à l'épitope du groupe sanguin B présente la structure ci-dessous :

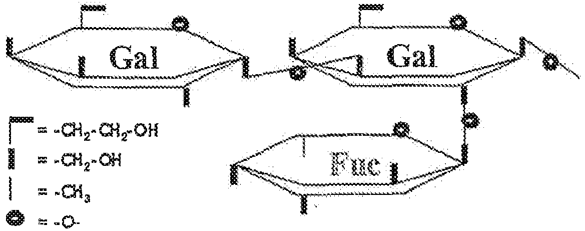

Avantageusement, le mélange de supports greffés de groupes antigéniquement similaires au groupe sanguin A et groupe sanguin B est en une proportion respective comprise entre 25/75 et 75/25 (v/-v). Il est en effet possible d'ajuster la proportion des deux supports dans la colonne à la population des donneurs selon la répartition des groupes sanguins de celle-ci. Dans le cadre d'une utilisation habituelle, la colonne sera remplie de préférence avec un mélange de 50/50 (v/v) en chaque support spécifique ci-dessus. On peut utiliser des colonnes analytiques de 15 à 25 cm de longueur et de 0,5 à 1 cm de diamètre. Dans le cas d'une mise en oeuvre à l'échelle pilote, on peut utiliser des colonnes de 40 à 60 cm de longueur et de 40 à 60 mm de diamètre. Dans ce cas, il est possible de charger la colonne de 600 ml de support d'immunoaffinité.

Un tel support est stocké en NaOH 1 M entre deux cycles d'utilisation. Avant utilisation, il est lavé par de l'eau.

On charge ensuite la colonne chromatographique d'immunoaffinité en concentré d'IgG de préférence à raison de 0,2 à 4 litres, en particulier de 1 à 2 litres, par millilitre de support. La spécificité d'un tel support ne nécessite pas un conditionnement préalable de la fraction d'IgG, c'est-à-dire que toute fraction ou concentré d'IgG obtenue par les techniques de fractionnement de plasma de l'art antérieur peut convenir.

La percolation du concentré ne fait pas intervenir le mécanisme d'élution. Par conséquent, quelle que soit la manière dont est obtenu le concentré d'IgG, il est percolé à travers la colonne, éventuellement grâce à une pompe. Cette percolation permet la rétention des AcaA et AcaB et des IgG polyréactives. Avantageusement, la colonne est ensuite lavée par de l'eau pour récupérer les IgG encore présentes dans le volume mort de la colonne.

Après percolation du concentré d'IgG, on obtient une fraction d'IgG appauvrie en AcaA et AcaB, ainsi qu'en IgG polyréactives issues du procédé de fabrication. En effet, les AcaA et AcaB sont retenus sur leur motif antigénique du support chromatographique qui en modifie la conformation. Par conséquent, les IgG polyréactives générées lors du procédé de fabrication sont également retenues sur les sites dévoilés par ce changement conformationnel. L'affinité de ces IgG polyréactives retenues de façon secondaire est beaucoup moins importante que celle des AcaA et AcaB. Il est possible de les éluer de manière fractionnée, après le passage des IgG, par l'utilisation d'un tampon d'élution contenant, par exemple, un sel de métal alcalino-terreux de concentration comprise entre 0,1 et 1,5 M, à un pH de 3-8,6.

Le procédé peut comprendre, après l'étape b), des étapes de concentration par ultrafiltration et de filtration stérilisante.

La colonne chromatographique et le support sont ensuite lavés avec une solution acide, telle que de glycine-HCl, pH 2,8, pour la désorption des AcaA et AcaB retenus sur le support. Ce support est ensuite rincé avec de l'eau et traité avec une solution de NaOH 1 M.

Le concentré d'IgG très appauvri en AcaA, AcaB et IgG polyréactives est ensuite soumis à une filtration d'élimination de virus résistants au traitement par solvant-détergent et/ou autres particules de taille supérieure à 20 nm, tels que les prions, les polymères d'IgG engendrés lors d'étapes de sa fabrication, les lipopolysaccharides en micelles, les acides nucléiques et les protéines agrégées. Un tel traitement représente avantageusement la nanofiltration, mise en oeuvre par des filtres de porosité décroisssante de 100 à 15 nm, en particulier sur trois filtres disposés en série et de seuils de rétention décroissants, de 100, 50 et 20 nm.

Le procédé peut comprendre, après l'étape c), une étape supplémentaire d'ajout de stabilisants afin, d'une part, d'assurer la stabilité des concentrés d'IgG en cours de conservation dans le temps et, d'autre part, permettre une lyophilisation évitant la dénaturation des IgG dans les diverses phases associées à celle-ci. De préférence, il sera ajouté une formulation stabilisante unique, pharmaceutiquement acceptable, répondant à l'objectif d'assurer la stabilisation des deux formes de conservation envisagées des IgG à la fois, à savoir sous forme liquide ou lyophilisée, et de conserver, voire améliorer, l'efficacité thérapeutique de ces IgG, comme décrit dans la demande de brevet WO 2004/091656 A2.

Selon d'autres modes de réalisation, il est également possible de récupérer sélectivement d'autres immunoglobulines, comme décrit dans la demande de brevet WO 02/092632 A1.

Les concentrés d'IgG sont éventuellement soumis à une étape ultérieure de concentration par ultrafiltration, puis à une filtration stérilisante et peuvent être conditionnés dans des flacons et conservés de préférence à des températures voisines de 4°C.

Comme largement explicité précédemment, les concentrés d'IgG selon l'invention comprennent des teneurs en AcaA et AcaB bien inférieures aux seuils admis par la Pharmacopée Européenne. Par conséquent, la méthode de dosage 2.6.20 (1997) qui y est indiquée, peut s'avérer insuffisamment sensible pour détecter les anticorps considérés présents en de très faibles teneurs dans les concentrés d'IgG de l'invention. Il est donc essentiel de mettre au point des méthodes de dosages de ces anticorps ayant un seuil de détection plus bas que celui du test ICP de la Pharmacopée Européenne appliqué à la détection des AcaA et AcaB.

Une telle méthode de dosage des anticorps anti-A et/ou anti-B dans les concentrés d'IgG de l'invention comprend les étapes consistant à :
a) préparer et calibrer une suspension d'hématies du groupe sanguin A, B et/ou O Rhésus +,
b) préparer des solutions d'anticorps anti-D monoclonal dans une plage de concentrations allant de 0 à 200 ng/ml dans un tampon biologiquement acceptable,
c) mettre en contact lesdites hématies avec des échantillons de solutions d'IgG ou avec les solutions d'anticorps anti-D monoclonal, et incuber les mélanges d'hématies ainsi obtenus pendant une durée prédéterminée,
d) ajouter dans chaque mélange d'hématies un fragment d'anticorps anti-IgG humaines F(ab')2 marqué au moyen d'un fluorochrome et incuber lesdites hématies,
e) soumettre chaque mélange d'hématies obtenu à l'étape d) à une cytométrie de flux,
f) déterminer la teneur en anticorps anti-A et/ou anti-B dans les concentrés d'IgG.

Un mode de mise en oeuvre d'une telle méthode de détermination de la teneur en anticorps anti-A et/ou anti-B peut comprendre la préparation d'une suspension d'hématies à 1% (v/v) du groupe sanguin A, B et/ou O dans un tampon PBS, de pH compris entre 7,0 et 7,4, contenant de 0,8 à 1,5 % en poids de sérum-albumine bovine BSA. Les hématies de la suspension sont comptées dans un dispositif de cytométrie de flux habituel, dont la mise en oeuvre est connue de l'homme du métier, puis de façon à calibrer la suspension à 37 à 43.10⁶ hématies/ml de suspension.

On prépare des solutions d'anticorps anti-D monoclonal, dont les concentrations sont comprises dans la plage allant de 0 à 200 ng/ml de tampon, de préférence un tampon PBS, de pH compris entre 7,0 et 7,4, contenant le cas échéant de 0,8 à 1,5 % en poids de sérum-albumine bovine BSA. Chaque solution ainsi préparée est dosée par absorptiométrie afin de déterminer son coefficient d'extinction molaire (ε).

Les concentrés d'IgG de l'invention sont ensuite ajustés à une concentration comprise dans la gamme de valeurs allant de 1 à 5 mg/ml, de préférence à 1 mg/ml, au moyen d'un tampon PBS, de pH compris entre 7,0 et 7,4, contenant de 0,8 à 1,5 % en poids de sérum-albumine bovine BSA.

On place un volume de 50 à 100 *µ*l de la suspension d'hématies de chaque groupe sanguin dans chaque puit d'une microplaque, par exemple de 96 puits, puis de 50 à 100 *µ*l de solutions d'IgG dans cette suspension d'hématies, ou de 50 à 100 *µ*l de solutions d'anticorps anti-D dans cette suspension d'hématies.

L'ensemble est mis à incuber pendant des durées comprises entre 1h30 et 2h30, en particulier 2h, à des températures habituellement comprises entre 30 et 40°C, de préférence 37°C.

Les différents mélanges d'hématies ainsi obtenus sont ensuite de préférence lavés avec le tampon PBS contenant de la BSA précédente et est centrifugées, puis on ajoute dans chaque mélange d'hématies, contenus dans un puit de microplaques, de 50 à 100 *µ*l d'anticorps F(ab')2 de chèvre anti-IgG humaines marquées d'un fluorochrome, tel que par exemple la phycoérythrine, présents dans du tampon PBS et de BSA défini précédemment.

L'incubation de l'ensemble est mise en oeuvre pendant environ 20-30 min à l'abri de la lumière.

Les différents mélanges d'hématies ainsi obtenus sont ensuite lavés et soumis à une cytométrie de flux mise en oeuvre avec tout appareil approprié disponible dans le commerce comportant un dispositif de détection de fluorescence des composés analysés.

L'intensité moyenne de fluorescence (IMF) est rapportée en fonction de la concentration en anticorps monoclonal anti-D et l'équation de la droite de régression est obtenue au moyen du logiciel Excel. Puis pour chaque échantillon, la concentration en équivalent anticorps anti-D est obtenue en utilisant l'équation linéaire de la droite de régression. Les échantillons ayant été dosés en triple, la moyenne de la concentration est établie et le coefficient de variation est calculé par le logiciel Excel.

On en déduit la teneur en anticorps anti-A et anti-B dans les concentrés d'IgG selon l'invention qui est celle avantageusement donnée précédemment.

De préférence, une méthode de dosage des AcaA et AcaB des concentrés d'IgG ci-dessus est effectuée par cytométrie de flux adaptée au contexte de l'invention, dont le principe repose sur l'utilisation d'hématies humaines de groupe A ou B, selon la détermination spécifique du titre des AcaA et AcaB voulue, mettant en oeuvre la détection d'un signal de fluorescence proportionnel à la teneur en ces anticorps.

Une telle méthode de dosage comprend les étapes consistant à :
a) préparer et calibrer une suspension d'hématies du groupe sanguin A ou B,
b) mettre en contact lesdites hématies avec des échantillons dilués de solutions d'IgG, et incuber le mélange ainsi obtenu pendant une durée prédéterminée,
c) incuber lesdites hématies en présence d'un anticorps anti-IgG marqué au moyen d'un fluorochrome, et
d) soumettre la suspension d'hématies obtenue à l'étape c) à une cytométrie de flux.

On prépare une suspension d'hématies à 1% (v/v) du groupe sanguin A ou B dans un tampon PBS, de pH compris entre 7,0 et 7,4, contenant de 0,8 à 1,5 % en poids de sérum-albumine bovine BSA. Les hématies de la suspension sont comptées dans un dispositif de cytométrie de flux habituel, dont la mise en oeuvre est connue de l'homme du métier, puis de façon à calibrer la suspension à 37 à 43.10⁶ hématies/ml de suspension.

On place un volume de 50 à 100 *µ*l de la suspension dans chaque puit d'une microplaque de 96 puits, puis de 50 à 100 *µ*l de différentes solutions d'IgG diluées de deux en deux à partir d'une solution de 30 g/l jusqu'à obtenir une solution d'IgG à 0,234 g/l.

L'ensemble est mis à incuber pendant des durées comprises entre 1h30 et 2h30, en particulier 2h, à des températures habituellement comprises entre 30 et 40°C, de préférence 37°C.

Les hématies sont ensuite lavées avec le tampon PBS contenant de la BSA précédente et est centrifugées puis on ajoute dans chaque puit de 50 à 100 *µ*l d'anticorps F(ab')₂ de chèvre anti-IgG humaines marquées d'un fluorochrome, tel que par exemple la phycoérythrine.

L'incubation de l'ensemble (l'étape c)) est mise en oeuvre pendant environ 20-30 min à l'abri de la lumière.

La suspension ainsi obtenue est ensuite lavée et soumise à une cytométrie de flux mise en oeuvre avec tout appareil approprié disponible dans le commerce comportant un dispositif de détection de fluorescence des composés analysés.

A titre d'exemple, les teneurs en anticorps anti-A et B de trois concentrés d'IgG dénommés B1, B2 et B3, préparés respectivement par fractionnement éthanolique selon la méthode de Cohn (citée plus haut) (B1), selon la demande de brevet WO 02/092632 (B2) et selon la demande de brevet WO 02/092632 suivie d'une chromatographie d'immunoaffinité (B3) pour l'appauvrissement en anticorps anti-A et anti-B, sont présentées dans le Tableau 1 qui suit. Les résultats sont présentés par rapport au titre témoin en anticorps anti-A et anti-B de l'échantillon B1 dont la teneur en ces anticorps a été fixée arbitrairement à 1, à titre de référence.

**Tableau 1**

| Echantillons | Titre en anticorps anti-A | Titre en anticorps anti-B |
|---|---|---|
| B1 | 1 | 1 |
| B2 | 3,65 | 3,85 |
| B3 | 0,68 | 0,52 |

Les résultats de ce tableau montrent tout d'abord que les teneurs en anticorps anti-A et anti-B des concentrés d'IgG (B1), préparés selon la méthode de Cohn, en contiennent environ quatre fois moins que les concentrés d'IgG (B2), préparés selon la méthode décrite dans WO 02/092632. De plus, le traitement ultérieur des ces concentrés d'IgG par des colonnes d'immunoaffinité spécifiques réduisent le titre en anticorps anti-A d'un facteur voisin de 5 et d'un facteur voisin de 7 en anticorps anti-B (B3).

Une autre méthode de détermination de la teneur en anticorps anti-A et anti-B pouvant être avantageusement mise en oeuvre consiste en la lyse par le complément in vitro, connue de l'homme du métier, mais qui a été spécifiquement mise au point pour les besoins de l'invention.

Une telle méthode de dosage comprend les étapes consistant à :
a) procéder à un radiomarquage d'une suspension d'hématies papaïnées choisies parmi les groupes sanguins A, B, AB et O, préalablement comptées, par un marqueur radioactif approprié,
b) mettre en contact les hématies radiomarquées avec des échantillons de concentrés d'IgG en un volume prédéterminé,
c) ajouter un volume identique à celui de l'étape b) de sérum normal du groupe sanguin AB,
d) incuber le mélange obtenu à l'étape c) pendant une durée prédéterminée, et
e) mesurer la radioactivité de la solution incubée ainsi obtenue.

On prépare une suspension d'hématies papaïnées à 1% (v/v) du groupe sanguin A, B, AB ou O qui est ensuite comptée dans une cellule de Malassez pour obtenir 10⁶ hématies. On ajoute 100 *µ*Ci de ⁵¹Cr (1 volume pour 1 volume d'hématies). On incube l'ensemble entre 1 et 2 heures, et les hématies radiomarquées sont ensuite lavées entre 4 et 6 fois.

Les hématies radiomarquées sont ensuite mises en contact avec des échantillons de concentrés d'IgG, à une concentration comprise de préférence entre 1 et 3 mg/ml, en particulier 1,2 mg/ml pour 4-6.10⁶ hématies radiomarquées, dans un volume par exemple de 100 *µ*l.

Un volume identique au précédent, par exemple de 100 *µ*l, de sérum normal de groupe sanguin AB est ensuite ajouté au mélange précédent pour apporter les différents facteurs de la voie de complément.

Le mélange réactionnel ainsi obtenu est ensuite incubé, de préférence pendant des durées comprises entre 3 et 5h, en particulier 4h, à des températures habituellement comprises entre 30 et 40°C, de préférence 37°C.

Le mélange réactionnel est ensuite, de préférence, centrifugé, et on procède à une mesure de la radioactivité de la solution incubée selon des dispositifs appropriés disponibles dans le commerce. La radioactivité mesurée de la solution est proportionnelle au taux d'hémolyse des hématies traitées et, par conséquent, à la teneur en anticorps anti-A et anti-B.

A titre d'exemple, les taux d'hémolyse des hématies des groupes sanguins A, B et AB obtenus en considérant un concentré d'IgG de l'invention (B3) et un concentré d'IgG de l'art antérieur (C1) ayant des taux d'hémolyse les plus faibles parmi les concentrés disponibles dans le commerce, sont indiqués au Tableau 2 qui suit.

**Tableau 2**

| Taux d'hémolyse d'hématies (%) | B3 | C1 (art antérieur) |
|---|---|---|
| Groupe A | 6 | 13 |
| Groupe B | 5 | 11 |
| Groupe AB | 6 | 13 |

Les exemples qui suivent illustrent des modes de réalisation de la présente invention sans toutefois en limiter la portée.

### Exemple 1

Un échantillon de concentré d'IgG à 40 g/l (B2) est obtenu par la mise en oeuvre du procédé décrit dans WO 02/092632.

Une colonne chromatographique de 50 cm de longueur et de 44 mm de diamètre est remplie d'un mélange 50/50 (v/v) de support GLYCOSORB ABO^{®} greffé par des trisaccharides correspondants aux épitopes du groupe sanguin A et du groupe sanguin B, puis est soumise à une étape préalable de lavage par 1200 ml d'eau.

On injecte du concentré d'IgG B2 à raison de 0,2 l/ml de support par l'intermédiaire d'une pompe. Une fois que ce volume a été percolé à travers la colonne, celle-ci est lavée par un volume minimal d'eau pour préparation injectable (PPI) afin de récupérer les IgG présentes dans le volume mort de la colonne.

On récupère un concentré d'IgG B3 à environ 40 g/l appauvri en AcaA, AcaB et en IgG polyréactives qui est soumis à une ultrafiltration de façon que le concentré soit à 60 g/l et à une nanofiltration d'élimination de virus sur trois filtres disposés en série et de seuils de rétention décroissants de 100, 50 et 20 nm.

La dissolution des excipients stabilisants constitués d'un mélange de glycine à 7 g/l, de mannitol à 30 g/l et de 20 ppm de Tween^{®}80 dans le concentré d'IgG à 60 g/l est suivie d'un ajustement de la concentration en IgG à 50 g/l au moyen d'eau PPI, puis le concentré est filtré stérilement et réparti en flacons.

### Exemple 2 : Quantification des anti-A/B dans les IgIV

### 1) Principe du dosage :

### 1-1) Préparation des hématies humaines

Les suspensions d'hématies humaines de groupe A rhésus +, B rhésus + ou O rhésus + sont normalisées à la concentration de 40 x 10⁶ hématies/ml dans du tampon PBS + 1 % BSA à pH = 7,4.

### 1-2) Préparation de la gamme d'anti-D monoclonal

Une préparation d'anti-D monoclonal (appelés R297) est dosée en Densité Optique (DO) à 280nm contre son tampon de PBS, de pH 7,4. Le coefficient d'extinction molaire (ε) de la protéine est calculé par rapport à sa composition en différents acides aminés et la concentration (C) en anti-D monoclonal est obtenue en appliquant la formule
C = DO/El avec l = largeur de la cuve pour effectuer la mesure de la DO.

Une gamme de 0 à 200 ng/ml d'anticorps monoclonal anti-D est réalisée en 12 points (200 ; 150 ; 100 ; 75 ; 50 ; 25 ; 12,5 ; 6,25 ; 3,13 ; 1,56 ; 0,78 et 0 ng/ml).

### 1-3) Préparation des solutions d'immunoglobulines

Différentes immunoglobulines intraveineuses, disponibles dans le commerce, ont été étudiées. Les caractéristiques principales de ces immunoglobulines sont détaillées dans le tableau ci-dessous :

| Désignation | Fournisseur | Concentration |
|---|---|---|
| Immunoglobuline polyvalente | A | 50 g/l |
| Immunoglobuline polyvalente | B | 50 g/l |
| Immunoglobuline humaine intraveineuse à 10% | C | 100 g/l |
| IgNG 2(*) | LFB | 50 g/l |
| IgNG 1 (**) | LFB | 50 g/l |
| TEGELINE^{®} | LFB | 50 g/l |

| | | |
|---|---|---|
| * réalisées au moyen de la mise en oeuvre du procédé décrit dans WO 02/092632 suivi de l'étape d'immunoaffinité décrite dans l'exemple 1 ** réalisées au moyen de la mise en oeuvre du procédé décrit dans WO 02/092632, sans étape d'immunoaffinité telle que décrite dans l'exemple 1 | | |

Les différentes préparations d'immunoglobulines sont ajustées à la concentration de 1 mg/ml au moyen d'un tampon PBS + 1 % BSA à pH=7,4.

### 1-4) Sensibilisation des hématies

Dans un micro plaque à fond rond, sont déposées dans les puits :
- 50 *µ*l de la suspension d'hématies A rhésus +, B rhésus + ou O rhésus + à 40 x 10⁶ hématies/ml,
- 50 *µ*l de la gamme anti-D ou 50 *µ*l des échantillons (IgIV) à doser.

Les échantillons à doser sont déposés en triple.

Les plaques sont ensuite incubées 2 heures à 37°C sous agitation.

### 1-5) lavages

Les plaques sont centrifugées 1 minute à 770 g. Le surnageant est écarté par retournement et puis 200 *µ*l de PBS + 1% BSA est ajouté dans chaque puits. L'opération est renouvelée 3 fois.

### 1-6) Ajout du conjugué et lavages

Un F(ab')2 de chèvre anti-IgG humaines (Fc spécifique) marqué à la phycoérythrine (PE) (Beckman Coulter Ref : PN IM0550) est dilué au 1/20^{ème} dans le tampon PBS + 1 % BSA pH=7,4 puis 50 *µ*l de la solution sont déposés dans chaque puits. La plaque est ensuite incubée 20 à 30 minutes à température ambiante à l'abri de la lumière. 3 lavages successifs sont réalisés tels que décrit au paragraphe 1-5).

### 1-7) Lecture au cytomètre de flux

Les suspensions d'hématies sont lues au cytomètre de flux (Beckman Coulter FC500) selon un programme approprié. La lecture est effectuée sur 50 000 évènements et l'appareil calcul automatiquement l'intensité moyenne de fluorescence (IMF) de chaque point de gamme ou échantillon.

### 1-8) Interprétation des résultats

L'IMF est rapportée en fonction de la concentration en anticorps monoclonal anti-D et l'équation de la droite de régression est obtenue au moyen du logiciel Excel. Puis pour chaque échantillon, la concentration en équivalent anticorps anti-D est obtenue en utilisant l'équation linéaire de la droite de régression. Les échantillons ayant été dosés en triple, la moyenne de la concentration est établie et le coefficient de variation est calculé par le logiciel Excel.

### 2) Résultats :

### 2-1) Concentration en anticorps anti-A

| Nom | Hématies O rhésus + | Hématies A rhésus + (ng Ig anti-A/mg d'Ig) | CV (%) |
|---|---|---|---|
| Immunoglobuline polyvalente, A | ns | 55,4 | 4,5 |
| Immunoglobuline polyvalente,B | ns | 44,4 | 3, 4 |
| Immunoglobuline humaine intraveineuse à 10%, C | ns | 117,9 | 14,6 |
| IgNG 2 | ns | 22,2 | 5,5 |
| IgNG 1 | ns | 119,8 | 4,9 |
| TEGELINE | ns | 35,6 | 5,0 |

| | | | |
|---|---|---|---|
| ns = non significatif | | | |

### 2-2) Concentration en anticorps anti-B

| Nom | Hématies O rhésus + | Hématies B rhésus + (ng Ig anti-B/mg d'Ig) | CV (%) |
|---|---|---|---|
| Immunoglobuline polyvalente, A | ns | 64,0 | 0,9 |
| Immunoglobuline polyvalente, B | ns | 42,4 | 5,1 |
| Immunoglobuline humaine intraveineuse à 10%, C | ns | 89,0 | 20,5 |
| IgNG 2 | ns | 16 | 9,9 |
| IgNG 1 | ns | 155,2 | 4,8 |
| TEGELINE | ns | 44,2 | 8,0 |

| | | | |
|---|---|---|---|
| ns = non significatif | | | |

### 3) Conclusions :

L'étape d'affinité est réellement contributive pour l'élimination des anticorps anti-A et anti-B. Parmi différentes immunoglobulines étudiées présentes sur le marché, le produit IgNG2 est le produit contenant le moins d'anticorps anti-A et d'anticorps anti-B.

### Exemple 3

On prépare une suspension d'hématies à 1% (v/v) du groupe sanguin A dans un tampon PBS, de pH 7,4, contenant 1% en poids de sérum-albumine bovine BSA. On prélève 50 *µ*l de la suspension d'hématies et on les introduit dans un tube pour un cytomètre de flux Beckmann-Coulter Epics XL, ainsi que 50 *µ*l d'une solution de marqueur interne mesurant le flux. La suspension est calibrée à 40.10⁶ hématies/ml.

Huit lots de solutions d'IgG sont préparés par dilution successive d'un facteur 2 du concentré d'IgG (v/v) (B3) obtenu à l'exemple 1, le lot le plus concentré étant à 30 g/l, le plus dilué à 0,234 g/l. On place ensuite un volume de 50 *µ*l de la suspension dans chaque puit d'une microplaque de 96 puits, puis 50 *µ*l des différentes solutions d'IgG diluées.

L'ensemble est mis à incuber pendant 2h à une température de 37°C, sous agitation.

Chaque puit est ensuite lavé avec 200 *µ*l de tampon PBS contenant de la BSA précédente et la microplaque est centrifugée pendant 1 minute à 2000 tours/min. Après élimination du surnageant, on ajoute dans chaque puit 50 *µ*l d'une solution diluée à 1/20 par du PBS-BSA d'anticorps F(ab')₂ de chèvre anti-IgG humaines marquées de fluorochrome de phycoérythrine (Beckmann Coulter).

L'incubation de l'ensemble est mise en oeuvre pendant 30 min à l'abri de la lumière.

La suspension ainsi obtenue est ensuite lavée comme précédemment.

Le culot de chaque puit est repris par 100 *µ*l de PBS-BSA. Le volume contenu dans chaque puit de la microplaque est transféré dans un tube dans lequel sont ensuite ajoutés 500 *µ*l de liquide de gaine Isoflow (Coulter) et est soumis à une cytométrie de flux mise en oeuvre avec le dispositif Coulter-Beckmann Epics XL comprenant des logiciels d'acquisition des données et d'exploitation des résultats. La fluorométrie est mesurée pour chaque échantillon.

On procède de la même manière avec des hématies de groupe sanguin B.

Ce mode opératoire est mis en oeuvre avec trois lots différents d'IgG (B3) et est également appliqué à trois lots différents d'IgG préparés par fractionnement éthanolique selon la méthode de Cohn (citée plus haut) (B1).

Les résultats obtenus figurent dans le Tableau 3 qui suit.

**Tableau 3**

| Echantillons | Titre en anticorps anti-A | Titre en anticorps anti-B |
|---|---|---|
| B1 | 1 | 1 |
| B2 (3 lots) | 3,80 ; 3,55 ; 3,59 | 3,80 ; 4,45 ; 3,31 |
| B3 (3 lots) | 0,66 ; 0,68 ; 0,69 | 0,33 ; 0,73 ; 0,50 |

### Exemple 4

On prépare une suspension d'hématies papaïnées à 1% (v/v) du groupe sanguin A, B, AB ou O qui est ensuite comptée dans une cellule de Malassez pour obtenir 10⁶ hématies. On ajoute 100 *µ*Ci de ⁵¹Cr (1 volume pour 1 volume d'hématies). On incube l'ensemble pendant 1 heure et les hématies radiomarquées sont ensuite lavées 5 fois.

Les hématies radiomarquées sont ensuite mises en contact avec des échantillons de concentrés d'IgG (B2) obtenus à l'exemple 1, à une concentration de 1,2 mg/ml pour 5.10⁶ hématies radiomarquées, dans un volume de 100 *µ*l.

Un volume identique au précédent de 100 *µ*l de sérum normal de groupe sanguin AB est ensuite ajouté au mélange précédent pour apporter les différents facteurs du complément.

Le mélange réactionnel ainsi obtenu est ensuite incubé, 4h, à une température de 37°C.

Le mélange réactionnel est ensuite centrifugé pendant 1 minute à 2000 tours/min, et on procède à une mesure de la radioactivité de la solution surnageante incubée, selon des dispositifs appropriés disponibles dans le commerce. La radioactivité mesurée de la solution est proportionnelle au taux d'hémolyse des hématies traitées et, par conséquent, à la teneur en anticorps anti-A et anti-B.

On procède de façon identique avec des hématies des groupes sanguins B, AB et O, étant tous de rhésus +, et avec un échantillon de sérum du groupe O+. Ce mode opératoire est mis en oeuvre avec trois lots différents d'IgG (B2).

En outre, on applique le procédé à trois lots d'échantillons du commerce de concentrés d'IgG, notés C2 à C4, et un échantillon C5 de sérum du groupe O+, inclus comme témoin négatif.

La radioactivité mesurée de la solution est proportionnelle au taux d'hémolyse des hématies traitées et, par conséquent, à la quantité en anticorps anti-A et anti-B fixés sur les hématies.

Les résultats d'hémolyse obtenus sont présentés dans le Tableau 4 suivant.

**Tableau 4**

| Taux d'hémolyse d'hématies (%) | B3 | C2 | C3 | C4 | C5 |
|---|---|---|---|---|---|
| | 6 | 16 | 13 | 30 | 34 |
| Groupe A+ | 6,2 | 15,5 | 13,5 | 31 | 34,4 |
| | 6,5 | 16,3 | 13,2 | 31 | 34,6 |
| Groupe B+ | 5 | 13,1 | 11.2 | 25 | 34 |
| | 4,8 | 13,3 | 10,8 | 25,3 | 34,4 |
| | 5,4 | 13,4 | 10,9 | 25,4 | 34,6 |
| Groupe AB+ | 6 | 16 | 15,1 | 31 | 34 |
| | 5,9 | 15,7 | 15,1 | 31,4 | 34,4 |
| | 6,5 | 16,3 | 15,4 | 30,9 | 34,6 |
| Groupe O+ | 0,1 | 0,2 | 0,22 | 0,33 | 2 |
| | 0,15 | 0,25 | 0,24 | 0,32 | 2,2 |
| | 0,12 | 0,21 | 0,24 | 0,30 | 2,7 |

Les résultats obtenus montrent que le concentré d'IgG B3 qui a été soumis à une chromatographie d'affinité selon l'invention contient la plus faible quantité en AcaA et AcaB étant donné que le taux d'hémolyse des hématies provenant des différents groupes sanguins est le plus faible.

On n'observe pas d'hémolyse avec des hématies de phénotype O+ inclus comme témoin négatif.

### Exemple 5

Mesure de la polyréactivité des concentrés d'IgG B2 (avant la chromatographie d'immunoaffinité) et après cette chromatographie (concentré d'IgG B3) décrite à l'exemple 1.

La mesure de la polyréactivité de ces concentrés d'IgG est effectuée selon le brevet EP 1 059 088 en utilisant deux antigènes qui réagissent avec les IgG polyréactives. Il s'agit de la myosine et de l'albumine modifiée par des groupes dinitrophényle (Albumine DNP).

Le Tableau 5 présente les facteurs d'enrichissement des IgG polyréactives des échantillons B2, B3 et C4 de l'exemple 3 dont la teneur en IgG a été fixée arbitrairement à 1, à titre de référence.

Ces mesures ont été effectuées sur trois lots différents de concentrés d'IgG considérés.

**Tableau 5**

| Echantillon | Myosine | Albumine DNP |
|---|---|---|
| B1 | 1 | 1 |
| B2 (3 lots) | 1,2 ; 0, 8 ; 1 2 | 3,2 ; 1,0 ; 2,0 |
| B3 (3 lots) | 0,4 ; 0,4 ; 0,4 | 1,0 ; 1,0 ; 1,0 |
| C4 (3 lots) | 2,0 ; 2,0 ; 2,0 | 8,0 ; 6,0 ; 7,0 |

Les résultats indiquent que le concentré d'IgG B3 de l'invention contient de 5 à 8 fois moins d'IgG polyréactifs que le concentré de l'art antérieur C4.

### Exemple 6

Exemple comparatif sur l'efficacité des concentrés IgG (B3) appauvries en AcaA, AcaB, et en IgG polyréactives, avec des concentrés d'IgG (B1).

L'étude a porté sur des souris déficientes en récepteurs FcγRI et FcγRIII traitées dans la perspective d'évaluation de l'activité immunomodulatrice des concentrés d'IgG selon l'invention. Ces animaux servent de modèle à un purpura thrombocytopénique.

On utilise comme témoin un concentré d'IgG (B1) obtenu par fractionnement éthanolique selon Cohn.

On applique le protocole expérimental décrit par Teeling J.L et al (Blood, 15/08/2001, vol. 98, number 4, pp.1095-1099).

Les plaquettes, effondrées par injection d'IgG monoclonales antiplaquettes de 9.10⁸/ml au niveau de 2.10⁸/ml, remontent à 7.10⁸/ml chez les animaux traités par les concentrés d'IgG B1 et B3 à une dose thérapeutique de 1 g/kg.

L'activité immunomodulatrice du concentré d'IgG B3 selon l'invention n'a pas été modifiée par la mise en oeuvre de la chromatographie d'immunoaffinité.

## Revendications

1. Concentré d'immunoglobulines G (IgG) à usage thérapeutique, **caractérisé en ce qu'**il présente des teneurs respectives en anticorps anti-A et anti-B conformes à un résultat négatif au test de Coombs indirect in vitro.

2. Concentré d'immunoglobulines G selon la revendication 1, comprenant une teneur en anticorps anti-A non supérieure à 23 ng/mg d'IgG, et une teneur en anticorps anti-B non supérieure à 20 ng/mg d'IgG.

3. Concentré d'immunoglobulines G selon la revendication 1 ou 2, présentant en outre une teneur d'IgG polyréactives résiduelle comprise entre 0,01% et 0,1%, en particulier entre 0,07 et 0,1%, par rapport à la teneur totale en IgG.

4. Concentré selon l'une des revendications 1 à 3, comprenant des stabilisants destinés à permettre le stockage dudit concentré.

5. Concentré selon les revendications 1 à 4, dans lequel les stabilisants représentent un mélange d'un sucre alcoolique, de préférence le mannitol ou sorbitol, de glycine et d'un détergent non ionique.

6. Concentré selon l'une des revendications 1 à 5, injectable par voie intraveineuse.

7. Procédé d'obtention d'un concentré d'IgG, comprenant les étapes suivantes de :
a) préparation d'un concentré d'IgG par fractionnement éthanolique et/ou par séparation chromatographique, associant une étape d'inactivation virale,
b) chromatographie d'immunoaffinité par percolation dudit concentré d'IgG sur un mélange de supports dont les matrices sont greffées de groupes oligosaccharides antigéniquement similaires aux groupes sanguins A et B, et
c) filtration d'élimination de virus et/ou de particules de taille supérieure à 20 nm.

8. Procédé selon la revendication 7, dans lequel l'étape a) du procédé de l'invention comprend une prépurification par précipitation de contaminants lipidiques à partir d'un plasma sanguin ou d'une fraction de plasma sanguin enrichie en IgG, une chromatographie unique sur un support de résine échangeuse d'anions effectuée à pH alcalin et une élution sélective des IgG en une étape par un tampon approprié à pH compris entre 4 et 7.

9. Procédé selon la revendication 7 ou 8, dans lequel les groupes oligosaccharides représentent des trisaccharides correspondants aux épitopes des groupes sanguins A et B.

10. Procédé selon la revendication 9, dans lequel les trisaccharides, correspondant à l'épitope du groupe sanguin A, présentent la structure N-acétylgalactosamine (GalNAc) - Galactose (Gal) - Fucose (Fuc), et ceux correspondant à l'épitope du groupe sanguin B, présentent la structure Galactose-Galactose-Fucose.

11. Procédé selon l'une des revendications 7 à 10, dans lequel l'étape d'inactivation virale est effectuée par solvant-détergent.

12. Procédé selon l'une des revendications 7 à 11, dans lequel le mélange de supports greffés de groupes antigéniquement similaires au groupe sanguin A et groupe sanguin B est en une proportion respective comprise entre 25/75 et 75/25 (v/v), de préférence 50/50 (v/v) en chacun desdits supports.

13. Procédé selon l'une des revendications 7 à 12, comprenant des étapes de concentration par ultrafiltration et de filtration stérilisante.

14. Procédé selon l'une des revendications 7 à 13, dans lequel la filtration d'élimination des virus est effectuée par nanofiltration.

15. Procédé selon l'une des revendications 7 à 14, comprenant, après l'étape c), une étape d'ajout de stabilisants de conservation audit concentré d'IgG.
